# EUROPÄISCHE PATENTSCHRIFT

(11) **EP 2 115 533 B1**
(45) Veröffentlichungstag und Bekanntmachung des Hinweises auf die Patenterteilung: **30.06.2010**
(21) Anmeldenummer: 07857097.5
(22) Anmeldetag: 21.12.2007
(51) Int. Cl.: G03C 1/73, C09K 9/02, C07D 311/78

(54) **PHOTOCHROME H-ANNELLIERTE BENZO(F)CHROMENE**
PHOTOCHROMIC H-ANNELLATED BENZO(F)CHROMENES
PHOTOCHROMES DE BENZO(F)CHROMÈNES H-ANNELÉS

(30) Priorität: 22.12.2006 DE 102006062280
(43) Veröffentlichungstag der Anmeldung: 11.11.2009
(73) Patentinhaber: Rodenstock GmbH, 80469 München (DE)
(72) Erfinder: MELZIG, Manfred, 82234 Wessling (DE); WEIGAND, Udo, 80638 München (DE); ROHLFING, Yven, 81547 München (DE)
(74) Vertreter: Müller-Boré & Partner Patentanwälte
(86) Internationale Anmeldenummer: PCT/EP2007/011390
(87) Internationale Veröffentlichungsnummer: WO 2008/077634

(56) Entgegenhaltungen:
- WO-A-02/22594
- WO-A-2006/045495

## Beschreibung

Die vorliegende Erfindung betrifft spezifische photochrome h-annellierte Benzo[f]chromen-Derivate sowie deren Verwendung in Kunststoffen aller Art, insbesondere für ophthalmische Zwecke. Insbesondere betrifft die vorliegende Erfindung von Benzo[f]chromenen abgeleitete photochrome Verbindungen, die weniger vergilben als im Stand der Technik verfügbare, vergleichbare Verbindungen, aber dennoch in der geschlossenen Form besonders langwellige Absorptionsmaxima bei gleichzeitig guter Leistungsfähigkeit in der offenen, farbigen Form aufweisen, wodurch sie bei Anwendung in phototropen Gläsern mit den darin verbreitet eingesetzten Indenonaphthopyranen gut harmonieren.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, dass diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Der Weltmarkt für photochrome Brillengläsern, sowohl aus Silikat wie aus Kunststoff, wird von den Farben grau und braun beherrscht. Färbungen wie grün, blau, magenta, orange oder gelb spielen eine völlig untergeordnete Rolle.

Bei allen derzeit auf dem Markt befindlichen photochromen Kunststoffgläsern werden diese beiden Farben durch Mischungen von mindestens zwei photochromen Farbstoffen erzielt. Diese können, wie in US 6,306,316 dargelegt, in zwei Gruppen eingeteilt werden, nämlich in solche, deren längstwelliges Absorptionsmaximum oberhalb von 550 nm liegt, die also im angeregten Zustand eine violette, blaue bis grüne Transmissionsfarbe ergeben, und in solche, deren längstwelliges Absorptionsmaximum unterhalb 550 nm liegt. Deren Transmissionsfarbe reicht von gelb über orange bis rot.

Zur ersten Gruppe zählen von 1-Naphtholen abgeleitete 2H-Naphthopyrane und ihre davon durch Annellierung abgeleiteten höheren analogen Derivate. Diese sind beispielsweise in US 5,698,141, US 5,723,072, US 6,146,554, US 6,225,466, US 6,331,625 bzw. US 6,340,765 beschrieben. Zu anderen Klassen gehörende photochrome Verbindungen, wie in US 4,931,220 oder EP 0 600 688 beschrieben, weisen zwar Absorptionsmaxima oberhalb von 550 nm auf, sind aber wegen ihrer geringen Lebensdauer bzw. der geringen Bandbreite der langwelligen Absorption nicht mehr im kommerziellen Einsatz. Die langwellig absorbierenden photochromen Farbstoffe aller derzeit auf dem Markt erhältlichen grauen oder braunen photochromen Kunststoffgläser (z.B. Rodenstock Perfalit ColorMatic Extra^{®} - seit 1999 -, Transitions Next Generation^{®} - seit 2002 -, Hoya Solio^{®} 1,55 - seit 2004) gehören zu den vorstehend von 1-Naphtholen abgeleiteten Verbindungen.

Zur zweiten Gruppe gehörende Farbstoffe sind meist in 2-Stellung Aryl- oder Heteroaryl substituierte 3H-Benzo- bzw. 3H-Napthopyrane, welche von 2-Naphtholen abgeleitet sind, wie in US 5,244,602, US 5,427,774, US 5,552,090, US 5,552,091, US 5,585,042 und WO 97/20239 beschrieben. Auch die in US 4,826,977 beschriebenen Spiroadamantan-substituierten Verbindungen gehören in diese Gruppe. Von 1-Naphtholen abgeleitete 2H-Naphtho[1,2-b]pyrane sind nur verwendbar, wenn die offene Form durch Substitution in 5-Stellung des Systems sterisch gehindert ist, wie in EP 1 248 778 beschrieben. Ohne diese Hinderung ist die Aufhellung für die Verwendung in Brillengläsern zu langsam.

Im Handel erhältliche photochrome Verbindungen, wie Reversacol Sunflower, Corn Yellow, Flame und Ruby (James Robinson) oder CNN-4 und CNN-8 (Tokuyama Soda) sind in EP 0 691 965 und US 6,719,926 beschrieben. Diese Verbindungen besitzen alle in 6-Stellung des Naphthopyransystems eine Aminogruppe, zumeist Piperidin oder Morpholin, die eine sehr hohe molare Extinktion (IOD > 1,5) im Absorptionsmaximum ergibt. Ohne diese funktionale Gruppe liegt der Wert niedriger. Leider hat dieses stark polare Substitutionsmuster eine ausgeprägte Solvatochromie zur Folge, so dass ein Teil des eingesetzten Farbstoffs in der festen Lösung (Kunststoffmatrix) in der offenen Form vorliegt. Dies zeigt sich in leicht farbigen Gläsern auch unter völligem Ausschluss von Anregungslicht. Zudem ist auch die Transmission nach V_{λ} um 4-10% herabgesetzt. Beispielhaft seien hier vor allem die braunen Varianten von Rodenstock ColorMatic Extra^{®} und Hoya Solio^{®} 1,55 genannt.

Ein weiterer Nachteil ist die im Vergleich zu den Farbstoffen der ersten Gruppe um 20-25 nm hypsochrom verschobene Absorption der geschlossenen Form. Die Abstimmung der Komposition, d.h. die jeweiligen Konzentrationen der eingesetzten photochromen Farbstoffe, zur Erzielung einer grauen oder braunen Farbe erfolgt derart, dass die Wunschfarbe im normalen direkten oder indirekten Sonnenlicht erreicht wird. Wird der ganz kurzwellige Anteil des sichtbaren Sonnenlichts (380-400 nm) selektiv gefiltert oder geblockt, z.B. durch wärmedämmend beschichtetes Fensterglas oder Verbundglas in Kraftfahrzeugen, so nehmen die Gläser im Falle eines grauen Glases eine blaue bzw. im Falle eines braunen Glases eine graue Farbe an. Dies kann in taghellen Räumen bei den heute am Markt erhältlichen photochromen Kunststoffgläsern gut beobachtet werden und ist ein kosmetischer Nachteil.

In WO 02/22594 sind Verbindungen beschrieben, die aufgrund ihrer Struktur zwar eine längerwellige Absorption der geschlossenen Form aufweisen. Der wesentliche Aspekt liegt hier jedoch in der Bereitstellung leistungsstarker langwellig absorbierender, also violetter bis blauer Farbstoffe, d.h. die eine längerwellige Absorption der offenen Form aufweisen. Dies wurde durch die Einführung von Aminosubstituenten in das Naphthopyransystem erreicht. Längstwellige Absorptionsmaxima unterhalb 540 nm sind auf diesem Wege nicht erreichbar. Ein Nachteil ist jedoch auch hier wieder die Vorfärbung bei der Verwendung in Brillenglas-Kunststoffmaterialien.

US 5,869,658 beschreibt Verbindungen ähnlicher Grundstruktur, deren offene Form im gewünschten Spektralbereich absorbiert. In dieser Schrift werden jedoch nur Indeno-annellierte Naphtho[2,1-b]pyrane behandelt, die zudem in 6-Stellung durch eine Alkoxygruppe substituiert sind. Dies ist durch die Synthese zwingend notwendig, da der Ringschluss ohne einen aktivierenden Substituenten, der die Verknüpfungsstelle in p-Position nucleophil macht, nicht stattfindet. Größere annellierte Ringsysteme als der 5-Ring sind nicht beschrieben und auf diesem Weg nicht möglich. Zudem sind die Aufhellgeschwindigkeiten sehr hoch. Dies führt bei Belichtung im Gleichgewicht nur zu einem geringen Anteil offener, d.h. farbiger Moleküle. Die Eindunkelungsleistung ist gering (ΔOD < 0,5). Ebenso ist die Absorption der geschlossenen Form mit Maxima zumeist unter 370 nm deutlich kürzerwellig als die der Verbindungen der ersten Gruppe. Dies ist jedoch nicht ausreichend, den langwelligen UV-Anteil des Sonnenlichts auszunutzen. Die in US 5,869,658 offenbarten Farbstoffmoleküle sind relativ planar, da die Abstoßung der H-Atome (in 8- und 9-Stellung des Formelbilds in Spalte 21) nicht sehr stark ist. Das Beispiel 3 fällt aus dem Rahmen, da hier die langsame Aufhellung und damit stärkere Eindunkelung ΔOD durch einen Fluor-Substituenten in 2-Stellung an den Phenylringen B bzw. B' erzwungen wird. Diese Wirkung, etwa eine Vervierfachung des ΔOD Werts, ist bereits in US 5,066,818 beschrieben. Diese die freie Rotation behindernde Substitution führt jedoch zu einer unerwünscht starken Abhängigkeit der Aufhellung von der das Molekül umgebenden Matrix, d.h. einer sehr breiten Verteilung der Aufhellgeschwindigkeit, wenn das Molekülensemble in einer Matrix betrachtet wird, bzw. einer stark unterschiedlichen Aufhellgeschwindigkeit in verschiedenen Kunststoffmaterialien.

EP 1 230 234 beschreibt ebenfalls 2H-Diarylnaphthopyrane, die mit ankondensierten Ringen substituiert sind. Die Kondensation mit einem Indenring in der gezeigten Struktur führt zu Verbindungen, die aufgrund der nicht vorhandenen sterischen Hinderung zwischen der CH₂-Gruppe des 5-Rings und dem H-Atom in 8-Stellung meist so schnell wie die Vergleichsverbindung C5 aufhellen. Verbindungen dieser Struktur sind, wie in US 3,567,605 beschrieben, nur bei sehr tiefen Temperaturen ausreichend photochrom. Auch beim 6-Ring ist die Hinderung noch sehr gering, die Aufhellung schnell und damit die beobachtete Einfärbung nur gering. Zudem ist die Auswahl möglicher Verbindungen sehr gering, die beschriebene Synthese lässt nur die Herstellung in 6- und 7-Stellung mit aktivierenden Gruppen substituierten Verbindungen zu. Bei Verwendung nur einer Methoxygruppe oder weniger aktivierenden höheren Alkoxygruppen findet keine Ringschlussreaktion statt. Größere Alkanringe sind synthetisch möglich, die sterische Hinderung ist jedoch im Vergleich zu den Verbindungen der Struktur gemäß US 5,869,658 oder WO 02/22594 immer gering, ebenso die Eindunkelungsleistung. Der wesentliche Aspekt dieser Verbindungen gemäß EP 1 230 234 war jedoch in erster Linie die Bereitstellung von intrinsisch grauen oder braunen Verbindungen. In der offenen Form steht die (substituierte) Phenylgruppe meta statt para zur E-thylenbrücke, dies führt zu völlig anderem, teilweise sogar kontroversem Verhalten.

In WO 2006/045495 werden spezifische photochrome h-annellierte Benzo[f]chromen-Derivate offenbart, die in der geschlossenen Form besonders langwellige Absorptionsmaxima bei gleichzeitig guter Leistungsfähigkeit in der offenen, farbigen Form aufweisen. Jedoch zeigen die darin offenbarten Verbindungen ein noch nicht zufriedenstellendes Vergilbungsverhalten.

Somit liegt der vorliegenden Erfindung die Aufgabe zugrunde, neue photochrome Farbstoffe bereitzustellen, die verbesserte Eigenschaften gegenüber den im Stand der Technik beschriebenen Verbindungen besitzen sollen. Insbesondere sollen die photochromen Verbindungen weniger vergilben als im Stand der Technik verfügbare, vergleichbare Verbindungen, aber dennoch in der geschlossenen Form besonders langwellige Absorptionsmaxima bei gleichzeitig guter Leistungsfähigkeit in der offenen, farbigen Form aufweisen.

Diese Aufgabe wird durch die in den Ansprüchen gekennzeichneten Gegenstände gelöst.

Insbesondere werden photochrome h-annellierte Benzo[f]chromene mit der allgemeinen Formel (I) bereitgestellt: 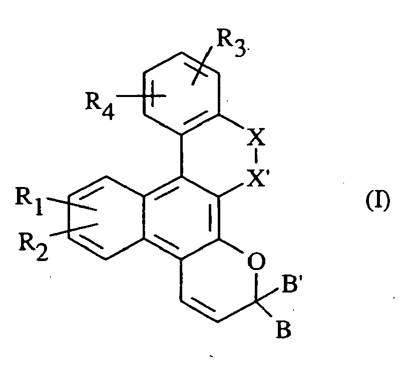 worin
X und X' jeweils unabhängig voneinander aus O, S, NR₅ oder CR₆R₇ ausgewählt sind, mit der Maßgabe, dass mindestens eine der Gruppen X oder X' durch CR₆R₇ dargestellt wird;
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₃-C₇)-Cycloalkylrest, der ein oder mehrere Heteroatome aufweisen kann, einem (C₁-C₆)-Alkoxyrest, einem (C₁-C₆)-Thioalkylrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können; oder
der Gruppe γ, worin die Reste R₁ und R₂ bzw. R₃ und R₄ jeweils eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann;
der Rest R₅ einen Substituenten darstellt, ausgewählt aus der Gruppe β oder aus einem Wasserstoffatom, einem (C₁-C₆)-Alkylrest oder einem (C₃-C₇)-Cycloalkylrest, der ein oder mehrere Heteroatome aufweisen kann;
oder der Rest R₅, wenn X für NR₅ steht, zusammen mit dem Rest R₃ des direkt benachbarten Benzoringes eine C₂-C₄-Kette bildet, die einen oder mehrere Substituenten aus der Gruppe α+β aufweisen können;
die Reste R₆ und R₇ jeweils unabhängig voneinander einen Substituenten darstellen, ausgewählt aus der Gruppe α und Phenyl, wobei R₆ und R₇ nicht beide -OH sein können;
oder die Reste R₆ und R₇, wenn X für CR₆R₇ steht, zusammen mit dem Rest R₃ des direkt benachbarten Benzoringes einen annellierten, un-, mono- oder disubstituierten Benzo- oder Pyridoring bilden, dessen Substituenten aus der Gruppe α und Phenyl ausgewählt sein können;
oder die Reste R₆ und R₇ zusammen einen (C₃-C₇)-Cycloalkylrest darstellen, der ein oder mehrere Heteroatome, wie beispielsweise Sauerstoff oder Schwefel, aufweisen kann, wobei an diesen Cycloalkylrest ein Benzoring annelliert sein kann;
B und B' unabhängig voneinander aus einer der folgenden Gruppen a), b), c) oder d) ausgewählt sind, wobei
a) mono-, di- und trisubstituierte Phenyl- oder Naphthylreste sind;
b) unsubstituierte, mono- und disubstituierte Heteroarylreste sind, wobei der Heteroarylrest Pyridyl, Furanyl, Benzofuran-2-yl, Benzofuran-3-yl, Dibenzo-furanyl, Thienyl, Benzothien-2-yl, Benzothien-3-yl oder Dibenzothienyl ist;
   wobei die Substituenten der Aryl- oder Heteroarylreste in a) und b) solche sind, ausgewählt aus den Gruppen α oder β, oder zwei direkt benachbarte Substituenten miteinander eine an den Benzoring gebundene -E-(CH₂)ₖ-F- Gruppe bilden können, worin k für 1 oder 2 steht und E und F unabhängig voneinander für Sauerstoff, Schwefel, CH₂, C(CH₃)₂ oder C(C₆H₅)₂ stehen und an diese -E-(CH₂)ₖ-F- Gruppe wiederum ein Benzoring annelliert sein kann;
   oder ausgewählt
   aus einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sind;
   oder ausgewählt
   aus einer der Gruppen N-Morpholin, N-Thiomorpholin, N-Piperidin, N-Azacycloheptan, N-Piperazin, N-(N'-(C₁-C₆)-Alkyl)piperazin, N-Pyrrolidin, N-Imidazolidin, N-Pyrazolidin, N-Aziridin, N-Azetidin, N-Indolin, N-Carbazol, N-Phenothiazin, N-Phenazin, N-Phenoxazin, N-Tetrahydrochinolin, die unsubstituiert sein können oder einen oder mehrere Substituenten aus der Gruppe α oder β aufweisen können;
c) Struktureinheiten mit den folgenden Formeln (II) und (III) sind: 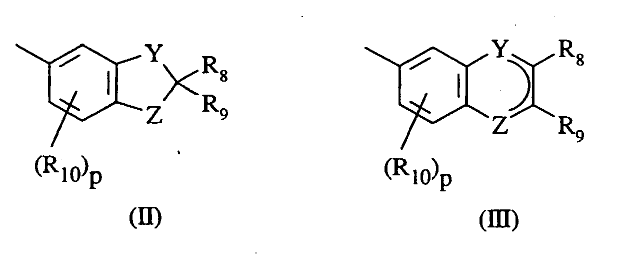 worin
   Y und Z unabhängig voneinander für O, S, CH₂, CMe₂, NH, N-Phenyl oder N(C₁-C₆)-Alkyl stehen, die Reste R₈ und R₉ unabhängig voneinander Wasserstoff oder einen (C₁-C₆)-Alkylrest darstellen und der Rest R₁₀ ein Substituent aus der Gruppe α ist, wobei p 1, 2, oder 3 ist;
d) Struktureinheiten mit den folgenden Formeln (IV) und (V) sind: 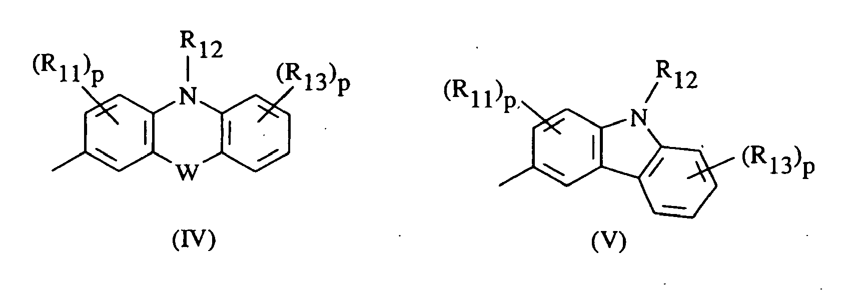
worin
W für O, S oder [C(R₆)(R₇)]_{g} mit g = 1, 2 oder 3 steht und R₆ und R₇ wie vorstehend definiert sind, R₁₁ und R₁₃ unabhängig voneinander aus der Gruppe α ausgewählt ist, wobei p 1, 2 oder 3 ist, und R₁₂ einen Substituenten darstellt, ausgewählt aus der Gruppe β oder aus einem Wasserstoffatom, einem (C₁-C₆)-Alkylrest und einem (C₃-C₇)-Cycloalkylrest, der ein oder mehrere Heteroatome aufweisen kann;
mit der Maßgabe, dass mindestens einer der Reste B oder B' aus d) ausgewählt ist.

In den erfindungsgemäßen Verbindungen gemäß Formel (I) ist der in h-Position des Benzo[f]chromen-Systems annellierte Cyclus bzw. Heterocyclus ein Sechsring.

Das Fünfringsystem mit einer CH₂-Brücke ist nahezu planar, aber stark verspannt. Mit dem Standard-Molekülgeometrie-Programm Hyperchem 7 (Monte Carlo, Mm+, 100 Zyklen) ergibt sich für den Winkel ε (Verdrehung des Phenylrings gegen die Naphthalinringebene) der Wert -0,27°. Die Einführung eines Sauerstoffatoms entspannt das Molekül durch Verdrehung des Winkels ε auf -27,25°. In gleicher Weise wirkt der Ersatz der CH₂-Brücke durch eine CH₂-CH₂-Brücke zum Sechsring (ε = -25,39°). Der Siebenring mit einer CH₂-CH₂-CH₂-Brücke führt bereits zu einer helicen-ähnlichen Struktur (ε = -49,25°).

Der in h-Position des Benzo[f]chromen-Systems annellierte Sechsring mit N, O oder S an der Stelle X hat deutlich stärkeren aromatischen Charakter als mit einer CH₂- oder CR₂-Gruppe. Dies führt zu einer bathochromen Verschiebung der Absorption.

Bevorzugte photochrome h-annellierte Benzo[f]chromen-Derivate gemäß der vorliegenden Erfindung weisen die nachfolgende allgemeine Formel (I) auf: 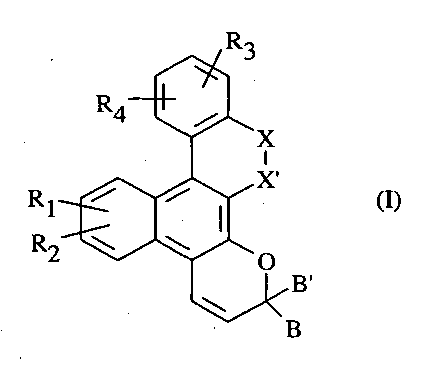 worin X, X', R₁, R₂, R₃, R₄, R₅, R₆ und R₇ wie vorstehend definiert sind und der aus d) gewählte Rest bezüglich B bzw. B' aus einem Carbazolyl-, (N-Methyl)-carbazolyl-, (N-Ethyl)-carbazolyl-, (N-Phenyl)-carbazolyl-, Phenoxazinyl-, (N-Methyl)-phenoxazinyl-, (N-Ethyl)-phenoxazinyl-, (N-Phenyl)-phenoxazinyl-, lminodibenzyl-, (N-Methyl)-iminodibenzyl-, (N-Ethyl)-iminodibenzyl- oder (N-Phenyl)-iminodibenzyl-Rest ausgewählt ist.

Die Reste R₁ und R₂ bzw. R₃ und R₄ können jeweils eine an den aromatischen Ring gebundene -A-(CH₂)ₖ-D- Gruppe mit k = 1 oder 2 bilden, wobei A und B unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann. Als -A-(CH₂)ₖ-D- Einheit kann insbesondere -O-(CH₂)₂-O-angeführt werden, wobei gegebenenfalls an die Ethylengruppe davon ein Benzocyclus annelliert ist. Die -A-(CH₂)ₖ-D- Einheit ist dabei über A und D bzw. E und F in ortho-Stellung zueinander an den jeweiligen Benzoring gebunden. Dies gilt auch für die vorstehende -E-(CH₂)ₖ-F- Einheit.

Vorzugsweise werden die Reste R₁, R₂, R₃ und R₄ gemäß der vorstehenden Formel (I) aus den Gruppen α und β ausgewählt.

Der Rest R₅ wird vorzugsweise aus einem (C₁-C₆)-Alkylrest, einem (C₃-C₇)-Cycloalkylrest, der ein oder mehrere Heteroatome aufweisen kann, und Phenyl ausgewählt.

Die Reste R₆ und R₇ werden jeweils unabhängig voneinander vorzugsweise aus der Gruppe α ausgewählt. X kann insbesondere O und CR₆R₇ sein, wobei die Reste R₆ und R₇ wie vorgenannt definiert sind. Wenn X für CR₆R₇ steht, können die Reste R₆ und R₇, wie beispielsweise Sauerstoff oder Schwefel, miteinander insbesondere für einen (C₃-C₇)-Cycloalkylrest stehen, der ein oder mehrere Heteroatome aufweisen kann.

In einer besonders bevorzugten Ausführungsform sind in den vorstehend aufgeführten Formeln (I) ,(II), (III) und (IV) B oder B' ein mono-, di- und trisubstituierter Arylrest, wobei der Arylrest jeweils ein Phenylrest oder ein Naphthylrest ist, während der andere Rest B bzw. B' aus d) ausgewählt ist.

Besonders bevorzugte photochrome h-annellierte Benzo[f]chromen-Derivate gemäß der vorliegenden Erfindung sind:
(1) 6-Methoxy-2-(4-methoxyphenyl)-2-(3-(N-methyl)-carbazolyl)-2H-13,14-dihydro-naphtho[1,2-h]-benzo[f]chromen
(2) 13-Methyl-2-(3-(N-methyl)-carbazolyl)-2-phenyl-2H-13,14-dihydro-naphtho[1,2-h]-benzo[f]chromen
(3) 13-Methyl-2-(3-(N-ethyl)-carbazolyl)-2-phenyl-2H-13,14-dihydro-naphtho[1,2-h]-benzo[f]chromen
(4) 2-(4-Methoxyphenyl)-2-(3-(N-phenyl)-carbazolyl)-2H-13,14-dihydro-naphtho[1,2-h] -benzo[f]chromen,
(5) 2-(4-Methoxyphenyl)-2-(3-(N-phenyl)-carbazolyl)-13-dimethyl-2H-13,14-dihydro-naphtho[1,2-h]-benzo[f]chromen
(6) 13-Methyl-2-phenyl-2-(3-(N-phenyl)-carbazolyl)-2H-13,14-dihydro-naphtho[1,2-h]-benzo[f]chromen
(7) 6-Methoxy-2-phenyl-2-(3-(N-methyl)-carbazolyl)-2H-13,14-dihydro-naphtho[1,2-h]-benzo[f]chromen
(8) 2-(4-Methoxyphenyl)-2-phenyl-2-(3-(N-phenyl)-carbazolyl)-13,14-tetramethyl-2H-13,14-dihydro-naphtho [1,2-h]-benzo[f]chromen
(9) 6-Phenyl-2-(4-methylphenyl)-2-(3-(N-phenyl)-phenoxazinyl)-2H-13,14-dihydro-naphtho[1,2-h]-benzo[f]chromen
(10) 6-Methoxy-2-phenyl-2-(3-(N-methyl)-iminodibenzyl)-2H-13,14-dihydro-naphtho-[1,2-h]-benzo[f]chromen und
(11) 13-Methyl-2-phenyl-2-(3-(N-phenyl)-iminodibenzyl)-2H-13,14-dihydro-naphtho-[1,2-h]-benzo[f]chromen.

Gemäß der vorliegenden Erfindung werden durch h-Annellierung von Benzo[f]chromen-Systemen Verbindungen bereitgestellt, deren photochrome Eigenschaften gegenüber den im Stand der Technik bekannten Verbindungen Vorzüge aufweisen. Insbesondere weisen die erfindungsgemäßen Verbindungen langwellige Absorptionsmaxima in der geschlossenen (farblosen) Form auf. Gleichzeitig zeigen die erfindungsgemäßen photochromen h-annellierten Benzo[f]chromen-Derivate gegenüber entsprechenden Verbindungen aus dem Stand der Technik vergleichbar gute Lebensdauer- und bessere Kinetikeigenschaften, d.h. schnelle, den heute verwendeten langwellig absorbierenden photochromen Farbstoffen vergleichbare Aufhellungsgeschwindigkeit sowie gutes Verhalten im Lebensdauertest. Insbesondere zeichnen sich die erfindungsgemäßen Benzo[f]chromene dabei durch ein verbessertes Verhalten hinsichtlich Vergilbung ("Yellowing") aus, wie anhand nachstehender Tabelle aufgezeigt.

| | | |
|---|---|---|
| | 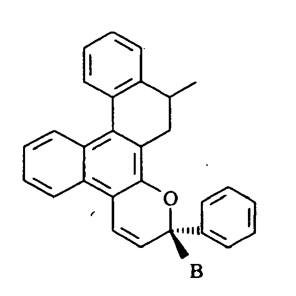 | 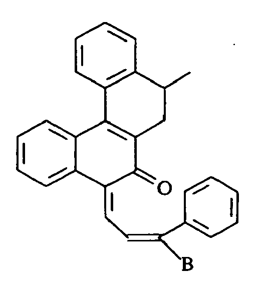 |
| | nicht angeregt (farblos) | angeregt (farbig) |
| Gruppe B | Vergilbung (nicht angeregt) Zunahme Gelbindex Δb* nach Lebensdauertest (2 mm Planglas; 500 ppm in Acrylat-Matrix | λₘₐₓ (angeregt) |
| 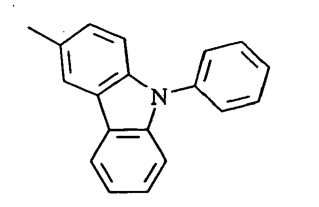 | Δb* = 4,3 | 480 nm |
| Verbindung (6) | | |
| 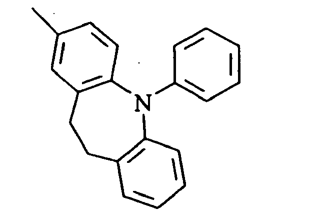 | Δb* = 5,5 | 450 nm |
| Verbindung (11) | | |
| 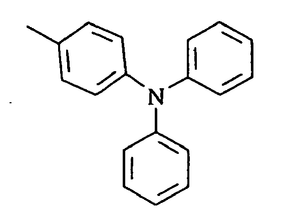 | Δb* = 15,5 | 540 nm |
| Stand der Technik | | |
| (WO 2006/045495) | | |

Zur Messung der Eigenschaften der erfindungsgemäßen photochromen Farbstoffe sowie der Verbindung aus dem Stand der Technik wurden jeweils 500 ppm des Farbstoffes in einer Acrylatmonomer-Matrix gelöst und nach Zusatz eines Polymerisations-Initiators mit Hilfe eines Temperaturprogrammes thermisch polymerisiert.

Die Transmissionseigenschaften der so hergestellten Kunststoffgläser (Dicke 2 mm) wurden anschließend nach DIN EN ISO 8980-3 vermessen. In der Tabelle aufgeführt ist der Unterschied des Gelbwertes b* des Glases im nicht angeregten Zustand vor und nach Lebensdauertest ("Vergilbung" während der Lebensdauer). Je kleiner dieser Wert, umso geringer ist die Vergilbung.

Der Lebensdauertest besteht aus einer ununterbrochenen 50-stündigen Bestrahlung mit Xenon-Bogenlampen in einem für diesen Zweck handelsüblichen Bestrahlungsgerät.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Benzo[f]chromen-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzo[f]chromen-Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Benzo[f]chromen-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen h-annellierten Benzo[f]chromen-Derivate beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Die erfindungsgemäßen photochromen h-annellierten Benzo[f]chromen-Derivate mit der allgemeinen Formel (I) lassen sich durch Umsetzung von geeignet substituierten annellierten 2-Naphthol-Derivate mit geeignet substituierten 2-Propin-1-ol-Derivaten in bekannter Weise (vgl. WO 02/22594) synthetisieren.

Die bei natürlicher oder künstlicher Alterung entstehenden Nebenprodukte der erfindungsgemäßen photochromen h-annellierten Benzo[f]chromen-Derivate mit der allgemeinen Formel (I) zeigen gegenüber vergleichbaren Verbindungen aus dem Stand der Technik deutlich geringere Absorption im kurzwelligen sichtbaren Teil des Spektrums.

## Patentansprüche

1. Photochrome h-annellierte Benzo[f]chromene mit der allgemeinen Formel (I): 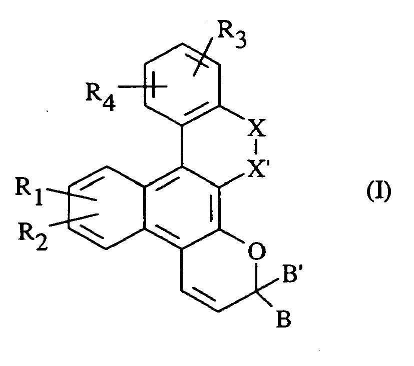 worin
X und X' jeweils unabhängig voneinander aus O, S, NR₅ oder CR₆R₇ ausgewählt sind, mit der Maßgabe, dass mindestens eine der Gruppen X oder X' durch CR₆R₇ dargestellt wird;
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₃-C₇)-Cycloalkylrest, der ein oder mehrere Heteroatome aufweisen kann, einem (C₁-C₆)-Alkoxyrest, einem (C₁-C₆)-Thioalkylrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können; oder
der Gruppe γ, worin die Reste R₁ und R₂ bzw. R₃ und R₄ jeweils eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann;
der Rest R₅ einen Substituenten darstellt, ausgewählt aus der Gruppe β oder aus einem Wasserstoffatom, einem (C₁-C₆)-Alkylrest oder einem (C₃-C₇)-Cycloalkylrest, der ein oder mehrere Heteroatome aufweisen kann;
oder der Rest R₅, wenn X für NR₅ steht, zusammen mit dem Rest R₃ des direkt benachbarten Benzoringes eine C₂-C₄-Kette bildet, die einen oder mehrere Substituenten aus der Gruppe α+β aufweisen können;
die Reste R₆ und R₇ jeweils unabhängig voneinander einen Substituenten darstellen, ausgewählt aus der Gruppe α und Phenyl, wobei R₆ und R₇ nicht beide -OH sein können;
oder die Reste R₆ und R₇, wenn X für CR₆R₇ steht, zusammen mit dem Rest R₃ des direkt benachbarten Benzoringes einen annellierten, un-, mono- oder disubstituierten Benzo- oder Pyridoring bilden, dessen Substituenten aus der Gruppe α und Phenyl ausgewählt sein können;
oder die Reste R₆ und R₇ zusammen einen (C₃-C₇)-Cycloalkylrest darstellen, der ein oder mehrere Heteroatome aufweisen kann, wobei an diesen Cycloalkylrest ein Benzoring annelliert sein kann;
B und B' unabhängig voneinander aus einer der folgenden Gruppen a), b), c) oder d) ausgewählt sind, wobei
a) mono-, di- und trisubstituierte Phenyl- oder Naphthylreste sind;
b) unsubstituierte, mono- und disubstituierte Heteroarylreste sind, wobei der Heteroarylrest Pyridyl, Furanyl, Benzofuran-2-yl, Benzofuran-3-yl, Dibenzo-furanyl, Thienyl, Benzothien-2-yl, Benzothien-3-yl oder Dibenzothienyl ist;
wobei die Substituenten der Aryl- oder Heteroarylreste in a) und b) solche sind, ausgewählt aus den Gruppen α oder β, oder zwei direkt benachbarte Substituenten miteinander eine an den Benzoring gebundene -E-(CH₂)ₖ-F- Gruppe bilden können, worin k für 1 oder 2 steht und E und F unabhängig voneinander für Sauerstoff, Schwefel, CH₂, C(CH₃)₂ oder C(C₆H₅)₂ stehen und an diese -E-(CH₂)ₖ-F- Gruppe wiederum ein Benzoring annelliert sein kann;
oder ausgewählt
aus einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sind;
oder ausgewählt
aus einer der Gruppen N-Morpholin, N-Thiomorpholin, N-Piperidin, N-Azacycloheptan, N-Piperazin, N-(N'-(C₁-C₆)-Alkyl)piperazin, N-Pyrrolidin, N-Imidazolidin, N-Pyrazolidin, N-Aziridin, N-Azetidin, N-Indolin, N-Carbazol, N-Phenothiazin, N-Phenazin, N-Phenoxazin, N-Tetrahydrochinolin, die unsubstituiert sein können oder einen oder mehrere Substituenten aus der Gruppe α oder β aufweisen können;
c) Struktureinheiten mit den folgenden Formeln (II) und (III) sind: 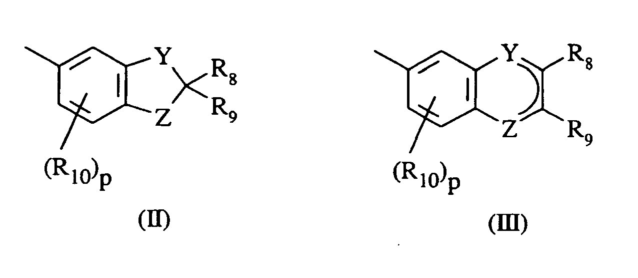 worin
Y und Z unabhängig voneinander für O, S, CH₂, CMe₂, NH, N-Phenyl oder N(C₁-C₆)-Alkyl stehen, die Reste R₈ und R₉ unabhängig voneinander Wasserstoff oder einen (C₁-C₆)-Alkylrest darstellen und der Rest R₁₀ ein Substituent aus der Gruppe α ist, wobei p 1, 2, oder 3 ist;
d) Struktureinheiten mit den folgenden Formeln (IV) und (V) sind: 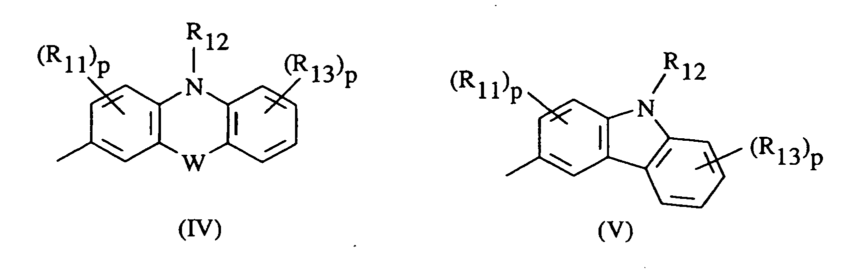
worin
W für O, S oder [C(R₆)(R₇)]_{g} mit g = 1, 2 oder 3 steht und R₆ und R₇ wie vorstehend definiert sind, R₁₁ und R₁₃ unabhängig voneinander aus der Gruppe α ausgewählt ist, wobei p 1, 2 oder 3 ist, und R₁₂ einen Substituenten darstellt, ausgewählt aus der Gruppe β oder aus einem Wasserstoffatom, einem (C₁-C₆)-Alkylrest und einem (C₃-C₇)-Cycloalkylrest, der ein oder mehrere Heteroatome aufweisen kann;
mit der Maßgabe, dass mindestens einer der Reste B oder B' aus d) ausgewählt ist.

2. Photochrome Benzo[f]chromene nach Anspruch 1, wobei die Reste R₁, R₂, R₃ und R₄ aus den Gruppen α und β ausgewählt sind.

3. Photochrome Benzo[f]chromene nach einem der Ansprüche 1 bis 2, wobei der Rest R₅ aus der Gruppe β oder aus einem Wasserstoffatom, einem (C₁-C₆)-Alkylrest, einem (C₃-C₇)-Cycloalkylrest, der ein oder mehrere Heteroatome aufweisen kann, oder Phenyl ausgewählt ist.

4. Photochrome Benzo[f]chromene nach einem der Ansprüche 1 bis 3, wobei die Reste R₆ und R₇ jeweils unabhängig voneinander aus der Gruppe α und Phenyl ausgewählt sind.

5. Photochrome Benzo[f]chromene nach einem der Ansprüche 1 bis 4, worin in der Formel (I) X, X', R₁, R₂, R₃, R₄, R₅, R₆ und R₇ wie vorstehend definiert sind und der aus d) gewählte Rest bezüglich B bzw. B' aus einem Carbazolyl-, (N-Methyl)-carbazolyl-, (N-Ethyl)-carbazolyl-, (N-Phenyl)-carbazolyl-, Phenoxazinyl-, (N-Methyl)-phenoxazinyl-, (N-Ethyl)-phenoxazinyl-, (N-Phenyl)-phenoxazinyl-, Iminodibenzyl-, (N-Methyl)-iminodibenzyl-, (N-Ethyl)-iminodibenzyl- oder (N-Phenyl)-iminodibenzyl-Rest ausgewählt ist.

6. Photochrome Benzo[f]chromene nach einem der vorhergehenden Ansprüche, welche
(1) 6-Methoxy-2-(4-methoxyphenyl)-2-(3-(N-methyl)-carbazolyl)-2H-13,14-dihydro-naphtho[1,2-h]-benzo[f]chromen
(2) 13-Methyl-2-(3-(N-methyl)-carbazolyl)-2-phenyl-2H-13,14-dihydro-naphtho[1,2-h]-benzo[f]chromen
(3) 13-Methyl-2-(3-(N-ethyl)-carbazolyl)-2-phenyl-2H-13,14-dihydro-naphtho[1,2-h]-benzo[f]chromen
(4) 2-(4-Methoxyphenyl)-2-(3-(N-phenyl)-carbazolyl)-2H-13,14-dihydro-naphtho[1,2-h] -benzo[f]chromen,
(5) 2-(4-Methoxyphenyl)-2-(3-(N-phenyl)-carbazolyl)-13-dimethyl-2H-13,14-dihydro-naphtho[1,2-h]-benzo[f]chromen
(6) 13-Methyl-2-phenyl-2-(3-(N-phenyl)-carbazolyl)-2H-13,14-dihydro-naphtho[1,2-h]-benzo[f]chromen
(7) 6-Methoxy-2-phenyl-2-(3-(N-methyl)-carbazolyl)-2H-13,14-dihydro-naphtho[1,2-h]-benzo[f]chromen
(8) 2-(4-Methoxyphenyl)-2-phenyl-2-(3-(N-phenyl)-carbazolyl)-13,14-tetramethyl-2H-13,14-dihydro-naphtho [1,2-h]-benzo[f]chromen
(9) 6-Phenyl-2-(4-methylphenyl)-2-(3-(N-phenyl)-phenoxazinyl)-2H-13,14-dihydro-naphtho[1,2-h]-benzo[f]chromen
(10) 6-Methoxy-2-phenyl-2-(3-(N-methyl)-iminodibenzyl)-2H-13,14-dihydro-naphtho-[1,2-h]-benzo[f]chromen und
(11) 13-Methyl-2-phenyl-2-(3-(N-phenyl)-iminodibenzyl)-2H-13,14-dihydro-naphtho-[1,2-h]-benzo[f]chromen
sind.

7. Verwendung der photochromen Berizo[f]chromene nach einem der Ansprüche 1 bis 6 in Kunststoffmaterialien.

8. Verwendung nach Anspruch 7, wobei das Kunststoffmaterial eine ophthalmische Linse ist.

## Claims

1. Photochromic h-annellated benzo[f]chromenes with the general formula (I): 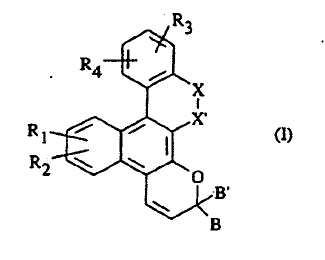 wherein
X and X', respectively independently of one another, are selected from O, S, NR₅ or CR₆R₇, on condition that at least one of the groups X or X' is represented by CR₆R₇;
the residues R₁, R₂, R₃ and R₄, respectively independently of one another, represent a substituent selected from
the group α consisting of one hydrogen atom, a (C₁-C₆) alkyl residue, a (C₃-C₇) cycloalkyl residue, which can have one or more heteroatoms, a (C₁-C₆) alkoxy residue, a (C₁-C₆) thioalkyl residue, a hydroxy group, a trifluoromethyl group, bromine, chlorine and fluorine;
the group β consisting of an un-, mono- or disubstituted phenyl-, phenoxy-, benzyl-, benzyloxy-, naphthyl- or naphthoxy residue, wherein the substituents can be selected from group α and phenyl; or
the group γ, wherein the residues R₁ and R₂ or R₃ and R₄ respectively form an -A-(CH₂)ₖ-D- group or -A-(C(CH₃)₂)ₖ-D- group bonded to the aromatic ring where k = 1 or 2, wherein A and D are selected independently of one another from oxygen, sulphur, CH₂, C(CH₃)₂ or C(C₆H₅)₂, and wherein a benzo ring can in turn be annellated onto this -A-(CH₂)ₖ-D- group;
the residue R₅ represents a substituent selected from group β or from a hydrogen atom, a (C₁-C₆) alkyl residue or a (C₃-C₇) cycloalkyl residue, which can have one or more heteroatoms;
or when X stands for NR₅, residue R₅ together with residue R₃ of the directly adjacent benzo ring forms a C₂-C₄ chain, which can have one or more substituents from group α+β;
the residues R₆ and R₇, respectively independently of one another, represent a substituent selected from group α and phenyl, wherein R₆ and R₇ cannot both be -OH; or when X stands for CR₆R₇, residues R₆ and R₇ together with residue R₃ of the directly adjacent benzo ring form an annellated, un-, mono- or disubstituted benzo or pyrido ring, the substituents of which can be selected from group α and phenyl; or residues R₆ and R₇ together represent a (C₃-C₇) cycloalkyl residue, which can have one or more heteroatoms, wherein a benzo ring can be annellated onto this cycloalkyl residue;
B and B' are selected independently of one another from one of the following groups a), b), c) or d), wherein
a) are mono-, di- and trisubstituted phenyl or naphthyl residues;
b) are unsubstituted, mono- and disubstituted heteroaryl residues, wherein the heteroaryl residue is pyridyl, furanyl, benzofuran-2-yl, benzofuran-3-yl, dibenzo-furanyl, thienyl, benzothien-2-yl, benzothien-3-yl or dibenzothienyl;
wherein the substituents of the aryl or heteroaryl residues in a) and b) are such selected from groups α or β, or two directly adjacent substituents can together form an -E-(CH₂)ₖ-F- group bound to the benzo ring, wherein k stands for 1 or 2 and E and F independently of one another stand for oxygen, sulphur, CH₂, C(CH₃)₂ or C(C₆H₅)₂ and a benzo ring can in turn be annellated onto this -E-(CH₂)ₖ-F- group;
or selected
from an un-, mono- or disubstituted amino group, wherein the amine substituents are selected from a (C₁-C₆) alkyl residue, a (C₃-C₇) cycloalkyl residue, a phenyl or benzyl residue that is unsubstituted or substituted with one or more substituents from group
α,
or selected
from one of the groups N-morpholine, N-thiomorpholine, N-piperidine, N-azacycloheptane, N-piperazine, N-(N'-(C₁-C₆)-alkyl) piperazine, N-pyrrolidine, N-imidazolidine, N-pyrazolidine, N-aziridine, A-azetidine, N-indoline, C-carbazole, N-phenothiazine, N-phenazine, N-phenoxazine, N-tetrahydroquinoline, which can be unsubstituted or can have one or more substituents from group α or β;
c) structural units with the following formulae (II) and (III) are: 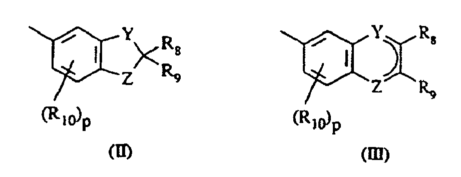 wherein
Y and Z, independently of one another, stand for O, S, CH₂, CMe₂, NH, N-phenyl or N(C₁-C₆) alkyl, residues R₈ and R₉, independently of one another, represent hydrogen or a (C₁-C₆) alkyl residue, and residue R₁₀ is a substituent from group α, wherein p is 1, 2 or 3;
d) structural units with the following formulae (IV) and (V) are: 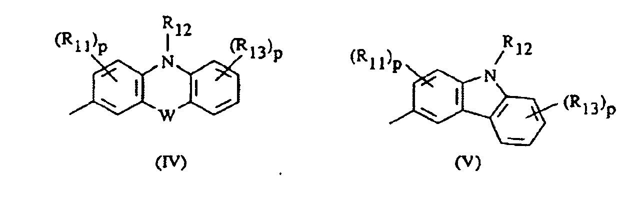
wherein
W stands for O, S or [C(R₆)(R₇)]_{g} with g = 1, 2 or 3 and R₆ and R₇ are as defined above, R₁₁ and R₁₃ are selected independently of one another from group α, wherein p is 1, 2 or 3, and R₁₂ represents a substituent selected from group β or from a hydrogen atom, a (C₁-C₆) alkyl residue and a (C₃-C₇) cycloalkyl residue, which can have one or more heteroatoms;
on condition that at least one of residues B or B' is selected from d).

2. Photochromic benzo[f]chromenes according to claim 1, wherein residues R₁, R₂, R₃ and R₄ are selected from groups α and β.

3. Photochromic benzo[f]chromenes according to one of claims 1 to 2, wherein residue R₅ is selected from group β or from a hydrogen atom, a (C₁-C₆) alkyl residue, a (C₃-C₇) cycloalkyl residue, which can have one or more heteroatoms, or phenyl.

4. Photochromic benzo[f]chromenes according to one of claims 1 to 3, wherein residues R₆ and R₇ are selected respectively independently of one another from group α and phenyl.

5. Photochromic benzo[f]chromenes according to one of claims 1 to 4, wherein in formula (I) X, X', R₁, R₂, R₃, R₄, R₅, R₆ and R₇ are as defined above and the residue selected from d) with respect to B or B' is selected from carbazolyl-, (N-methyl)-carbazolyl-, (N-ethyl)-carbazolyl-, (N-phenyl)-carbazolyl-, phenoxazinyl-, (N-methyl)-phenoxazinyl-, (N-ethyl)-phenoxazinyl-, (N-phenyl)-phenoxazinyl-, iminodibenzyl-, (N-methyl)-iminodibenzyl-, (N-ethyl)-iminodibenzyl- or (N-phenyl)-iminodibenzyl residue.

6. Photochromic benzo[f]chromenes according to one of the preceding claims, which are
(1) 6-methoxy-2-(4-methoxyphenyl)-2-(3-(N-methyl)-carbazolyl-2H-13,14-dihydro-naphtho[1,2-h]-benzo[f]chromene
(2) 13-methyl-2-(3-(N-methyl)-carbazolyl)-2-phenyl-2H-13,14-dihydro-naphtho[1,2-h]-benzo[f]chromene
(3) 13-methyl-2-(3-(N-ethyl)-carbazolyl)-2-phenyl-2H-13,14-dihydro-naphtho[1,2-h]-benzo[f]chromene
(4) 2-(4-methoxyphenyl)-2-(3-(N-phenyl)-carbazolyl)-2H-13, 14-dihydro-naphtho[1,2-h]-benzo[f]chromene
(5) 2-(4-methoxyphenyl)-2-(3-(N-phenyl)-carbazolyl)-13-dimethyl-2H-13,14-dihydro-naphtho[1,2-h]-benzo[f]chromene
(6) 13-methyl-2-phenyl-2-(3-(N-phenyl)-carbazolyl)-2H-13,14-dihydro-naphtho[1,2-h]-benzo[f]chromene
(7) 6-methoxy-2-phenyl-2-(3-(N-methyl)-carbazolyl)-2H-13,14-dihydro-naphtho[1,2-h]-benzo[f]chromene
(8) 2-(4-methoxyphenyl)-2-phenyl-2-(3-(N-phenyl)-carbazolyl)- 13,14-tetramethyl-2H-13,14-dihydro-naphtho[1,2-h]-benzo[f]chromene
(9) 6-phenyl-2-(4-methylphenyl)-2-(3-(N-phenyl)-phenoxazinyl)-2H-13,14-dihydro-naphtho[1,2-h]-benzo[f]chromene
(10) 6-methoxy-2-phenyl-2-(3-(N-methyl)-iminodibenzyl)-2H-13,14-dihydro-naphtho [1,2-h]-benzo[f]chromene, and
(11) 13-methyl-2-phenyl-2-(3-(N-phenyl)-iminodibenzyl)-2H-13,14-dihydro-naphtho[1,2-h]-benzo[f]chromene.

7. Use of the photochromic benzo[f]chromenes according to one of claims 1 to 6 in plastic materials.

8. Use according to claim 7, wherein the plastic material is an ophthalmic lens.

## Revendications

1. Photochromes de benzo[f]chromènes H-annelés de formule générale (I) : 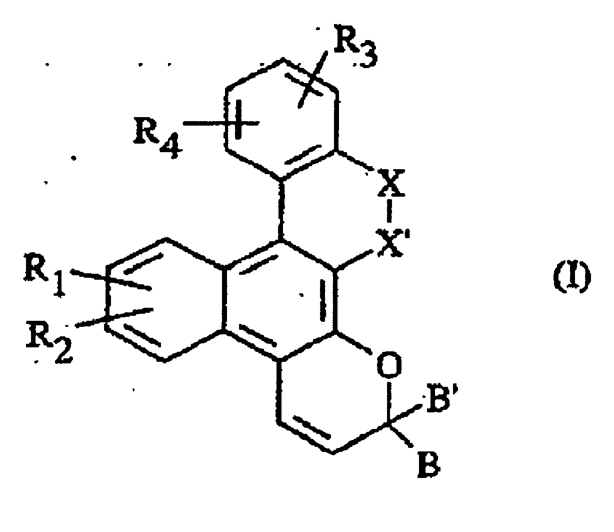 où :
X et X' sont, chacun indépendamment l'un de l'autre, choisis parmi 0, S, NR₅ ou CR₆R₇, sous réserve qu'au moins l'un des groupes X ou X' soit représenté par CR₆R₇;
les radicaux R₁, R₂, R₃ et R₄ représentent, chacun indépendamment les uns des autres, un substituant, choisi dans
le groupe α, comprenant un atome d'hydrogène, un radical alkyle (C₁-C₆), un radical cycloalkyle (C₃-C₇), qui peut présenter un ou plusieurs hétéroatomes, un radical alkoxy (C₁-C₆),un radical thioalkyle (C₁-C₆),un groupe hydroxy, un groupe trifluorométhyle, du brome, du chlore et du fluor;
le groupe β, comprenant un radical phényle, phénoxy, benzyle, benzyloxy, naphthyle ou naphthoxy non substitué, monosubstitué ou disubstitué, les substituants pouvant être choisis parmi le groupe α et phényle; ou
le groupe γ, dans lequel les radicaux R₁ et R₂ ou R₃ et R₄ forment respectivement un groupe -A-(CH₂)ₖ-D- ou -A-(C(CH₃)₂)ₖ-D lié au cycle aromatique, où k = 1 ou 2, A et D étant choisis, chacun indépendamment l'un de l'autre, parmi oxygène, soufre, CH₂, C(CH₃)2 ou C(C₆H₅)₂, et un cycle benzo pouvant lui-même être annelé à ce groupe -A-(CH₂)ₖ-D-;
le radical R₅ représente un substituant, choisi dans le groupe β, ou parmi un atome d'hydrogène, un radical alkyle (C₁-C₆) ou un radical cycloalkyle (C₃-C₇), qui peut présenter un ou plusieurs hétéroatomes;
ou le radical R₅ , lorsque X représente NR₅, forme avec le radical R₃ du cycle benzo directement adjacent, une chaîne C₂-C₄, qui peut présenter un ou plusieurs substituants du groupe α+β;
les radicaux R₆ et R₇ représentent, chacun indépendamment l'un de l'autre, un substituant, choisi parmi le groupe α et phényle, R₆ et R₇ ne pouvant pas être tous les deux -OH;
ou les radicaux R₆ et R₇, lorsque X représente CR₆R₇, forment avec le radical R₃ du cycle benzo directement adjacent, un cycle benzo ou pyrido annelé, non substitué, monosubstitué ou disubstitué, dont les substituants peuvent être choisis parmi le groupe α et phényle;
ou les radicaux R₆ et R₇ représentent ensemble un radical cycloalkyle (C₃-C₇) qui peut présenter un ou plusieurs hétéroatomes, un cycle benzo pouvant être annelé à ce radical cycloalkyle;
B et B' sont choisis, indépendamment l'un de l'autre, dans l'un des groupes a), b), c) ou d) ci-après,
a) étant des radicaux phényle ou napthtyle monosubstitués, disubstitués ou trisubstitués;
b) étant des radicaux hétéroaryle non substitués, monosubstitués et disubstitués, le radical hétéroaryle étant du pyridyle, furanyle, benzofurane-2-yle, benzofurane-3-yle, dibenzofuranyle, thiényle, benzothiène-2-yle,benzothiène-3-yle ou dibenzothiényle;
les substituants des radicaux aryle ou hétéroaryle dans a) et b) étant tels que choisis dans les groupes α ou β, ou bien deux substituants directement adjacents forment ensemble un groupe -E-(CH₂)ₖ-F-, dans lequel k est égal à 1 ou 2, et E et F représentent indépendamment l'un de l'autre de l'oxygène, du soufre, CH₂, C(CH₃)₂ ou C(C₆H₅)₂, et un cycle benzo peut lui-même être annelé à ce groupe -E-(CH₂)ₖ-F-;
ou choisis dans
un groupe amine non substitué, monosubstitué ou disubstitué, les substituants aminés étant choisis parmi un radical alkyle (C₁-C₆) , un radical cycloalkyle (C₃-C₇), un radical phényle ou benzyle non substitué ou substitué avec un ou plusieurs substituants du groupe α;
ou choisis dans
l'un des groupes N-morpholine, N-thiomorpholine, N-pipéridine, N-azacycloheptane, N-pipérazine, N-(N'-(C₁-C₆)-alkyle)pipérazine, N-pyrrolidine, N-imidazolidine, N-pyrazolidine, N-aziridine, N-azétidine, N-indoline, N-carbazole, N-phénothiazine, N-phénazine, N-phénoxazine, N-tétrahydrochinoline, qui peuvent être non substitués ou peuvent présenter un ou plusieurs substituants du groupe α ou β;
c) étant des unités structurales de formules (II) et (III) ci-après : 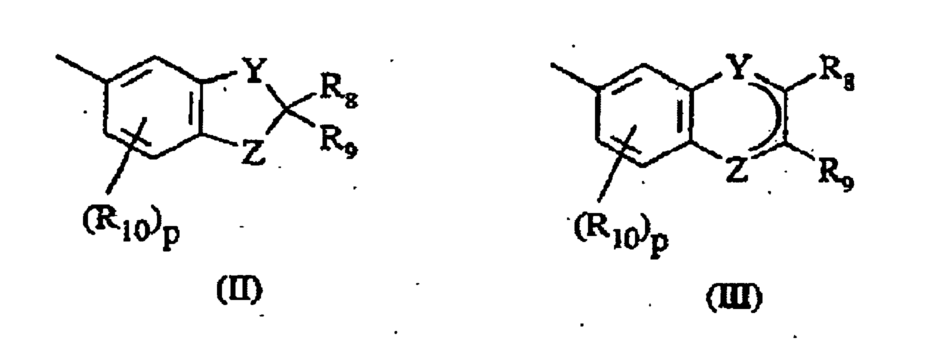 où :
Y et Z représentent, indépendamment l'un de l'autre, O, S, CH₂, Cme₂, NH, N-phényle ou N(C₁-C₆)-alkyle, les radicaux R₈ et R₉, représentent, indépendamment l'un de l'autre, de l'hydrogène ou un radical alkyle (C₁-C₆) et le radical R₁₀ est un substituant du groupe α, p étant égal à 1, 2 ou 3;
d) étant des unités structurales de formules (IV) et (V) ci-après : 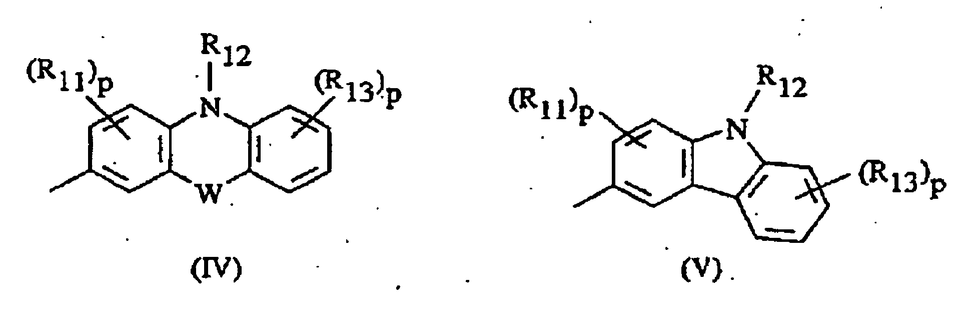
où :
W représente O, S ou [C(R₆) (R₇)]_{g}, où g = 1, 2 ou 3, et R₆ et R₇ sont définis comme précédemment, R₁₁ et R₁₃ sont choisis indépendamment l'un de l'autre dans le groupe α, p étant égal à 1, 2 ou 3, et R₁₂ représente un substituant, choisi dans le groupe β ou parmi un atome d'hydrogène, un radical alkyle (C₁-C₆) et un radical cycloalkyle (C₃-C₇) qui peut présenter un ou plusieurs hétéroatomes;
Sous réserve qu'au moins l'un des radicaux B ou B' soit choisi dans d).

2. Photochromes de benzo[f]chromènes selon la revendication 1, dans lesquels les radicaux R₁, R₂, R₃ et R₄ sont choisis dans les groupes α et β.

3. Photochromes de benzo[f]chromènes selon l'une des revendications 1 à 2, dans lesquels le radical R₅ est choisi dans le groupe β ou parmi un atome d'hydrogène, un radical alkyle (C₁-C₆) et un radical cycloalkyle (C₃-C₇) qui peut présenter un ou plusieurs hétéroatomes, ou phényl.

4. Photochromes de benzo[f]chromènes selon l'une des revendications 1 à 3, dans lesquels les radicaux R₆ et R₇ sont choisis, chacun indépendamment l'un de l'autre, parmi le groupe α et phényle.

5. Photochromes de benzo[f]chromènes selon l'une des revendications 1 à 4, dans lesquels, dans la formule (I), X, X', R₁, R₂, R₃, R₄, R₅, R₆ et R₇ sont définis comme précédemment et le radical choisi dans d), relativement à B ou B', est choisi parmi des radicaux carbazolyle,(N-méthyle)-carbazolyle,(N-éthyle)-carbazolyle, (N-phényle-carbazolyle, phenoxazinyle, (N-méthyle)-phénoxazilyne, (N-éthyle)-phénoxazilyne, N-phényle)-phénoxazilyne, iminodibenzyle, (N-méthyle)- iminodibenzyle, (N-éthyle)-iminodibenzyle ou (N-phényle)- iminodibenzyle.

6. Photochromes de benzo[f]chromènes selon l'une des revendications précédentes, qui sont
(1) 6-méthoxy-2-(4-méthoxyphényle)-2-(3-(N-méthyl)-carbazolyle)-2H-13,14-dihydro- naphtho[1,2-h]-benzo[f]chromes
(2) 13-méthyl-2-(3-(N-méthyl)-carbazolyle)-2-phényle-2H-13,14-dihydro-naphtho[1,2-h]-benzo[f]chromes
(3) 13-méthyl-2-(3-(N-éthyl)-carbazolyle)-2-phényle-2H-13,14-dihydro-naphtho[1,2-h]-benzo[f]chromes
(4) 2-(4-méthoxyphényle)-2-(3-(N-phényle)-carbazolyle)-2H-13,14-dihydro-naphtho[1,2-h]-benzo[f]chromes
(5) 2-(4-méthoxyphényle)-2-(3-(N-phényle)-carbazolyle)-13-diméthyl-2H-13,14-dihydro-naphtho[1,2-h]-benzo[f]chromes
(6) 13-méthyl-2-phényle-2-(3-(N-phényle)-carbazolyle-2H-13,14-dihydro-naphtho[1,2-h]-benzo[f]chromes
(7) 6-méthoxy-2-phényle-2-(3-(N-méthyl)-carbazolyle)-2H-13,14-dihydro-naphtho[1,2-h]-benzo[f]chromes.
(8) 2-(4-méthoxyphényle)-2-phényle-2-(3-(N-phényle)- carbazolyle)-2H-13,14-dihydro-naphtho[1,2-h]-benzo[f]chromes
(9) 6-phényle-2-(4-méthylphényle)-2-(3(N-phényle)-phénoxazinyle)-2H-13,14-dihydro-naphtho[1,2-h]-benzo[f]chromes.
(10) 6-méthoxy-2-phényle-2-(3-(N-méthyl)-iminodibenzyle)-2H-13,14-dihydro- naphtho[1,2-h]-benzo[f]chromes.
(11) 13-méthyl-2-phényle-2-(3-(N-phényle)-iminodibenzyle)-2H-13,14-dihydro-naphtho[1,2-h]-benzo[f]chromes.

7. Utilisation des photochromes de benzo[f]chromènes selon l'une des revendications 1 à 6 dans des matières plastiques.

8. Utilisation selon la revendication 7, la matière plastique étant une lentille ophtalmique.
